(19) **Europäisches Patentamt**
**European Patent Office**
**Office européen des brevets**

(11) **EP 1 661 909 B1**

(12) # EUROPEAN PATENT SPECIFICATION

(45) Date of publication and mention
of the grant of the patent:
**16.09.2009 Bulletin 2009/38**

(51) Int Cl.:
*A61K 38/55* (2006.01)    *A61K 38/05* (2006.01)
*A61K 38/06* (2006.01)    *A61P 9/12* (2006.01)
*C07K 5/06* (2006.01)    *C07K 5/08* (2006.01)

(21) Application number: **04771089.2**

(22) Date of filing: **30.07.2004**

(86) International application number:
**PCT/JP2004/010929**

(87) International publication number:
**WO 2005/012334 (10.02.2005 Gazette 2005/06)**

(54) **BIOLOGICALLY NON-DEGRADABLE PEPTIDE, ANGIOTENSIN CONVERTING ENZYME INHIBITOR, DRUG AND FUNCTIONAL FOOD**

BIOLOGISCH NICHT-ABBAUBARES PEPTID, INHIBITOR DES ANGIOTENSINUMWANDELNDEN ENZYMS, ARZNEIMITTEL UND FUNKTIONELLES NAHRUNGSMITTEL

PEPTIDE BIOLOGIQUEMENT NON BIODEGRADABLE, INHIBITEUR DE L'ENZYME DE CONVERSION DE L'ANGIOTENSINE, MEDICAMENT ET ALIMENT FONCTIONNEL

(84) Designated Contracting States:
**AT BE BG CH CY CZ DE DK EE ES FI FR GB GR HU IE IT LI LU MC NL PL PT RO SE SI SK TR**
Designated Extension States:
**LT LV**

(30) Priority: **01.08.2003 JP 2003285007**

(43) Date of publication of application:
**31.05.2006 Bulletin 2006/22**

(73) Proprietor: **Calpis Co., Ltd.**
**Shibuya-ku,**
**Tokyo 150-0022 (JP)**

(72) Inventors:
• **YAMAMOTO, N.,**
**Calpis Co., Ltd., Food Res. Lab.**
**Kanagawa 2290006 (JP)**
• **MIZUNO, S.,**
**Calpis Co., Ltd.**
**Shibuya-ku, Tokyo 150-0022 (JP)**
• **NISHIMURA, S.**
**deceased (JP)**
• **GOTOU, T.,**
**Calpis Co., Ltd.**
**Shibuya-ku, Tokyo 150-0022 (JP)**
• **MATSUURA, K.,**
**Calpis Co., Ltd., Food & Drink Development Lab.**
**Kanagawa 229006 (JP)**

(74) Representative: **Hallybone, Huw George et al**
**Carpmaels & Ransford**
**43-45 Bloomsbury Square**
**London WC1A 2RA (GB)**

(56) References cited:
**JP-A- 2003 024 012**

• **JOCHEN HEINS ET AL.: "KINETIC INVESTIGATION OF THE HYDROLYSIS OF AMINOACYL p-NITROANILIDES BY DIPEPTIDYL PEPTIDASE IV FROM HUMAN AND PIG KIDNEY" BIOCHIMICA ET BIOPHYSICA ACTA, vol. 785, no. 1-2, 28 February 1984 (1984-02-28), pages 30-35, XP002388026**
• **HANS G BOMAN ET AL.: "CHEMICAL SYNTHESIS AND ENZYMIC PROCESSING OF PRECURSOR FORMS OF CECROPINS A AND B" THE JOURNAL OF BIOLOGICAL CHEMISTRY, vol. 264, no. 10, 5 April 1989 (1989-04-05), pages 5852-5860, XP002388027**
• **KATO T ET AL: "SUCCESSIVE CLEAVAGE OF N-TERMINAL ARG1-PRO2 AND LYS3-PRO4 FROM SUBSTANCE P BUT NO RELEASE OF ARG1-PRO2 FROM BRADYKININ, BY X-PRO DIPEPTIDYL-AMINOPEPTIDASE" BIOCHIMICA ET BIOPHYSICA ACTA, AMSTERDAM, NL, vol. 525, no. 2, 1978, pages 417-422, XP008054073 ISSN: 0006-3002**

**(Cont. next page)**

- MOUSUMI GHOSH ET AL.: "CHARACTERIZATION OF NATIVE AND RECOMBINANT FORMS OF AN UNUSUAL COBALT-DEPENDENT PROLINE DIPEPTIDASE (PROLIDASE) FROM THE HYPERTHERMOPHILIC ARCHAON Pyrococcus furiosus" JOURNAL OF BACTERIOLOGY, vol. 180, no. 18, September 1998 (1998-09), pages 4781-4789, XP002388028
- LONDON R E ET AL: "13C nuclear magnetic resonance srudy the cis-trans isomerism in X-Pro-Pro tripeptides" BIOCHEMISTRY, AMERICAN CHEMICAL SOCIETY. EASTON, PA, US, vol. 17, no. 12, 13 June 1978 (1978-06-13), pages 2277-2283, XP002995615 ISSN: 0006-2960
- K KARIYA ET AL.: "INHIBITION OF ANGIOTENSIN CONVERTING ENZYME OF RAT BRAIN WITH BRADYKININ AND ITS FRAGMENTS" BIOCHEMICAL AND BIOPHYSICAL RECEARCH COMMUNICATIONS, vol. 100, no. 1, 15 May 1981 (1981-05-15), pages 31-36, XP008066005
- HONG-SON CHEUNG ET AL.: "BINDING OF PEPTIDE SUBSTRATES AND INHIBITORS OF ANGIOTENSIN-CONVERTING ENZYME" THE JOURNAL OF BIOLOGICAL CHEMISTRY, vol. 255, no. 2, 25 January 1980 (1980-01-25), pages 401-407, XP002388030
- MAKOTO SATAKE ET AL.: "TRANSEPITHELIAL TRANSPORT OF THE BIOACTIVE TRIPEPTIDE, VAL-PRO-PRO, IN HUMAN INTESTINAL CACO-2 CELL MONOLAYERS" BIOSCIENCES, BIOTECHNOLOGY, BIOCHEMISTRY, vol. 66, no. 2, February 2002 (2002-02), pages 378-384, XP002388031
- BALA M. ET AL.: 'Novel peptidomimics as angiotensin-converting enzyme inhibitors: a combinatorial approach' BIOORG.MED.CHEM. vol. 10, 2002, pages 3685 - 3691, XP002298550
- HELLBERG S. ET AL.: 'Minimum analogue peptide sets (MAPS)for quantitative structure-activity relantionships' INT.J.PEPT.PROTEIN RES. vol. 37, 1991, pages 414 - 424, XP000652502
- GOMAZKOV Q.A. ET AL.: 'Biochemical and physiological activity of novel peptide inhibitors of angi' BYULL.EKSP.BIOL.MED. vol. 222, 1996, pages 279 - 281, XP002995613
- HARRIS R.B. ET AL.: 'Dipeptide-hydroxamates are good inhibitors of the angiotensin I-converting enzyme' BIOCHEM.BIOPHYS.RES.COMMUN. vol. 116, 1983, pages 394 - 399, XP002995614
- LONDON R.E. ET AL.: '13C nuclear magnetic resonance srudy the cis-trans isomerism in X-Pro-Pro tripeptides' BIOCHEMISTRY vol. 17, 1978, pages 2277 - 2283, XP002995615
- ICHIMURA T. ET AL.: 'Angiotensin I-converting enzyme inhibitory activity and insulin secretion stimulative activity of fermented fish sauce' JOURNAL OF BIOSCIENCE AND BIOENGINEERING vol. 96, December 2003, pages 496 - 499, XP002995616

**Description**

The present invention relates to the use in medicine of peptides that are highly absorbable and hardly digestible in living organism when administered orally or through other route, and in particular their use as angiotensin converting enzyme inhibitors or in functional foods, such as foods for specified health use, having hypotensive effect.

A variety of peptides have been reported having various functions, such as angiotensin converting enzyme (abbreviated as ACE hereinbelow) inhibitory activity, hypotensive effect, anti-bacterial activity, calcium solubilizing effect, and immunomodulating effect, and these peptides are in use in food and medicine.

ACE converts a precursor, angiotensin I, to angiotensin II having vasoconstrictive activity in living organism, to thereby raise the blood pressure. Thus peptides having ACE inhibitory activity are expected to exhibit hypotensive effect by inhibiting ACE to suppress production of angiotensin II in living organism. Such peptides having ACE inhibitory activity have been reported in many publications including the following Patent Publications 1 to 3.

As peptides that are absorbed through the alimentary canal into blood to express their functions in living organism, *in vivo* indigestible peptides are expected to be advantageous that have high absorbability and digestion resistance against various digestive enzymes in living organism. For example, Patent Publication 4 teaches that lowmolecular weight peptides mainly composed of dipeptides and tripeptides and having an average chain length of not longer than 3, have excellent intestinal absorbability. However, it is not known in detail which peptides contribute to enhancement of the *in vivo* digestion resistance.

Thus development of peptides are desired that have high absorbability and digestion resistance in living organism, and are capable of effectively exhibiting useful functions, such as ACE inhibitory activity, in living organism.

Patent Publication 1: JP-2-62828-A
Patent Publication 2: JP-3-120225-A
Patent Publication 3: JP-6-40944-A
Patent Publication 4: JP-5-252979-A

Furthermore, Cheung et al "Binding of peptide substrates and inhibitors of angiotensin-converting enzyme. Importance of the COOH-terminal dipeptide sequence" J Biol Chem. 1980 Jan 25;255(2):401-7 reports an assay of several compounds, including the dipeptides Ile-Pro and Arg-Pro, as ACE inhibitors.

Harris et al "Dipeptide-hydroxamates are good inhibitors of the angiotensin I-converting enzyme" Biochem Biophys Res Commun- 1983 Oct 31;116(2):394-9 discloses the inhibition constants and modes of inhibition of a series of dipeptide-hydroxamate compounds, including Glu-Pro, using bovine lung parenchyma angiotensin I-converting enzyme.

It is an object of the present invention to provide for use in medicine *in vivo* indigestible peptides that are highly absorbable and hardly digestible in living organism when administered orally or through other route, and expected to effectively exhibit functions such as hypotensive effect in living organism.

It is another object of the present invention to provide for use in medicine as an angiotensin converting enzyme inhibitor, *in vivo* indigestible peptides that are highly absorbable and hardly digestible in living organism, and effectively exhibiting angiotensin I converting enzyme inhibitory activity in living organism.

It is still another object of the present invention to provide for use in medicine in a functional food *in vivo* indigestible peptides that are highly absorbable and hardly digestible in living organism, and effectively exhibiting hypotensive effect in living organism.

The present inventors have made intensive researches to find out that, among dipeptides and tripeptides having good *in vivo* absorbability, peptides having dipeptide Xaa-Pro or tripeptide Xaa-Pro-Pro sequences (Xaa may be any amino acid) with Pro at the carboxyl terminals, are indigestible in living organism, and capable of effectively exhibiting their functions in living organism. The inventors have also found out that, among the dipeptides and tripeptides having such sequences, those having particular sequences are particularly excellent in ACE inhibitory activity or the like properties, to thereby complete the present invention.

According to the present invention, there is provided a peptide having Pro at a carboxyl terminal, selected from the group consisting of Gln-Pro, Met-Pro, and Ser-Pro-Pro or a salt thereof, for use in medicine.

According to the present invention, there is also provided the peptide, or salt thereof, for use in medicine as an angiotensin converting enzyme inhibitor.

According to the present invention, there is further provided the peptide, or salt thereof, for use in medicine in a functional food having hypotensive effect.

According to the present invention, there is also provided the use of a peptide having Pro at a carboxy terminal, selected from the group consisting of Gln-Pro, Met- Pro, and Ser-Pro-Pro, or a salt thereof, in the manufacture of a functional food or medicament for reducing blood pressure.

Since the peptides of the present invention are dipeptides or tripeptides of particular sequences with Pro at the carboxyl terminals, the present peptides are highly absorbable and hardly digestible in living organism, and expected to effectively exhibit functions, such as hypotensive effect, in living organism.

The peptides of the present invention are also expected to effectively exhibit ACE inhibitory activity in living organism.

Since the medicament and functional food of the present invention contain the peptides of the present invention, the present medicament and functional food are expected to effectively exhibit hypotensive effect in living organism.

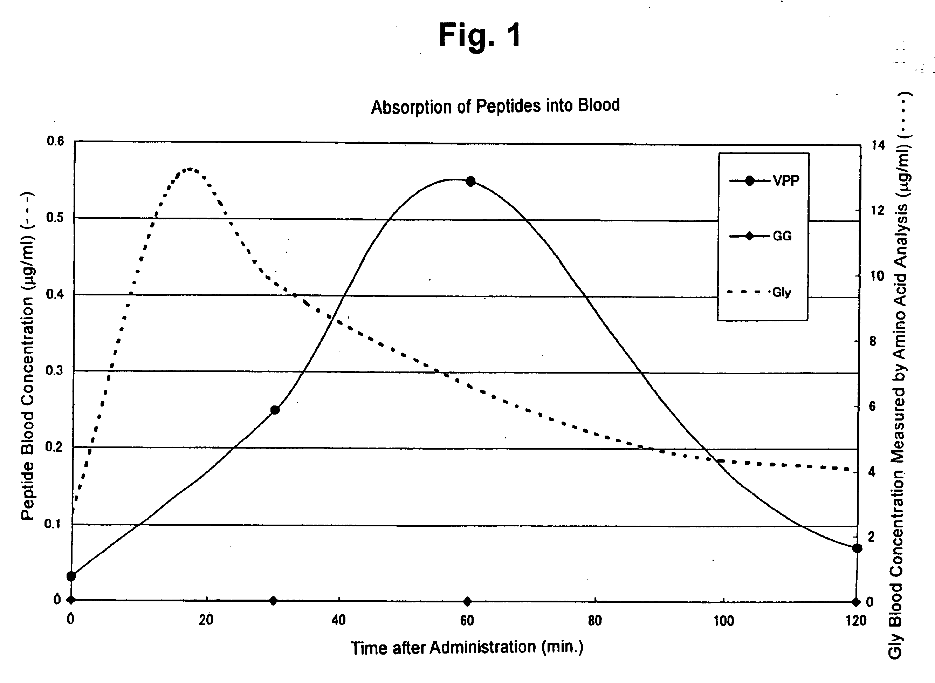

Fig. 1 is a graph showing the results of evaluation of *in vivo* absorbability and digestion resistance of Xaa-Pro and Xaa-Pro-Pro performed in Referential Example 1.

The present invention will now be explained in detail.

The peptide of the present invention is a dipeptide or tripeptide having Pro at the carboxyl terminal, consisting of at least one of Gln-Pro, Met-Pro, and Ser-Pro-Pro.

The peptide has high digestion resistance against *in vivo* peptidases when absorbed intestinally in living organism.

The peptide of the present invention may be prepared, for example, from the corresponding amino acids by ordinary organic synthesis or the like method. Alternatively, the present peptide may also be prepared, for example, by digesting food protein, such as animal milk casein, through fermentation with lactic acid bacteria, followed by purification; or by hydrolyzing food protein through an enzymatic method with an appropriate combination of proteinases and peptidases, followed by purification.

The purification does not have to result in isolation of the dipeptide or tripeptide, and may be concentration and purification by a suitable combination of conventional purification methods for increasing the peptide concentration.

The animal milk casein may be, for example, casein from cow's milk, horse's milk, goat's milk, and sheep's milk, with cow's milk casein being particularly preferred.

The peptides of the present invention may be used as salts thereof.

The peptide salt is preferably a pharmaceutically acceptable salt, such as salts of inorganic acid including hydrochloride, sulfate, or phosphate, or of organic acid including acetate, trifluoroacetate, citrate, maleate, fumarate, lactate, or tartrate.

The peptides of the present invention may optionally be combined with various auxiliary additives for improving the nutritional balance or flavor. Examples of such auxiliary additives may include various carbohydrates, lipids, vitamins, minerals, sweeteners, flavoring agents, pigments, and texture improvers.

The amount of the peptides of the present invention for use may suitably be selected depending on the kinds of peptides and salts thereof, and is not particularly limited. For example, when the peptides are in a functional food or the like for regular use by human, the amount of peptides may preferably be about 0.01 to 100 mg/kg per ingestion, with no particular upper limit.

For use in a medicament having hypotensive effect, the peptides of the present invention may be formulated with a pharmaceutically acceptable carrier into various dosage forms in accordance with conventional methods. For example, for solid formulation for oral administration, the present peptides may be mixed with a vehicle, and optionally a binder, disintegrator, lubricant, coloring agent, taste corrigent, flavor corrigent, or the like as desired, and formulated into tablets, coated tablets, granules, powders, capsules, or the like.

Such a medicament may optionally contain other components such as peptides having ACE inhibitory activity or hypotensive effect other than the present peptides.

The peptides of the present invention may be used by mixing in functional food such as foods for specified health use claiming hypotensive effect. Examples of such functional foodmay include beverages, yogurt, liquid food, jelly, candies, retort food, tablet candies, cookies, sponge cakes, bread, biscuits, and chocolates. The peptides may also be formulated into capsules or tablets for use as dietary supplements.

The present invention will now be explained in more detail with reference to Example.

Example 1

Among peptides having the sequence Xaa-Pro or Xaa-Pro-Pro present in casein derived from cow's milk, Ile-Pro, Glu-Pro, Arg-Pro, Gln-Pro, Met-Pro, and Ser-Pro-Pro were chemically synthesized (manufactured by TORAY RESEARCH CENTER, INC., not lower than 95 % purity). Solutions of each of these peptides at various concentrations were prepared, and measured for the ACE inhibitory activity in accordance with the method discussed below. The peptide concentration ($\mu$M) at which the ACE inhibitory effect was 50% was taken as IC50 value. The results are shown in Table 1.

<Evaluation of ACE Inhibitory Activity>

ACE derived from bovine lung (manufactured by WAKO PURE CHEMICAL INDUSTRIES, LTD.) was dissolved in a 0. 1M borate buffer at pH 8.3 in an amount of 0.1 U, to obtain an ACE solution. 80 $\mu$l of a diluted solution prepared by properly diluting a 5 mg/ml solution of each peptide shown in Table 1 with distilled water according to the IC50 value of that peptide, 200 μl of a 5 mM hippuryl-histidyl-leucine (manufactured by SIGMA) solution containing 300 mM NaCl, and 20 μl of the ACE solution prepared above were introduced into a tube, and reacted at 37°C for 30 minutes. Subsequently, the reaction was terminated by adding 250 μl of 1N hydrochloric acid (manufactured by WAKO PURE CHEMICAL INDUSTRIES, LTD.). 1.7 ml of ethyl acetate (manufactured by WAKO PURE CHEMICAL INDUSTRIES, LTD.) was then added, and stirred. 1.4 ml of the ethyl acetate layer was taken, placed in another tube, and evaporated at 120 °C for about 60 minutes to obtain a dried product. The dried product was dissolved in 1 ml of distilled water, and the absorbance at 228 nm of hippuric acid extracted with ethyl acetate was measured. As controls, absorbance was measured of a solution without the diluted solution and a solution without the diluted solution and the ACE solution. From the obtained absorbance, ACE inhibitory activity was calculated in accordance with the following formula.

$$\texttt{ACE inhibitory activity (\%) = [(A-B)/A] × 100}$$

A: (Absorbance of solution without diluted solution but with ACE solution) - (Absorbance of solution without diluted solution and ACE solution)

B: (Absorbance of solution with diluted solution and ACE solution) - (Absorbance of solution with diluted solution but without ACE solution)

Table 1

| Peptide sequence | IC50 value (μM) |
|---|---|
| Ile-Pro * | 443.9 |
| Glu-Pro* | 174.7 |
| Arg-Pro* | 275.2 |
| Gln-Pro | 65.8 |
| Met-Pro | 135.3 |
| Ser-Pro-Pro | 44.5 |
| * - COMPARATIVE EXAMPLE | |

Referential Example 1

<Evaluation of Absorbability and Digestion Resistance of Xaa-Pro and Xaa-Pro-Pro in Living Organism>

In order to evaluate the absorbability and digestion resistance in living organism of the peptides having the sequence Xaa-Pro or Xaa-Pro-Pro present in casein, the absorption of the peptides into blood after oral administration was tested in rats as follows.

To two six-week-old SD (Sprague Dawley) rats, 500 mg/animal each of Val-Pro-Pro as an example of Xaa-Pro-Pro and Gly-Gly as a dipeptide having no Pro were administered orally. Blood samples were taken from the portal vein at intervals, and the absorption of each peptide into blood was determined. The results are shown in Fig. 1.

From Fig. 1, it was confirmed that Gly-Gly was easily digested *in vivo*, so that Gly was detected, while Val-Pro-Pro was absorbed into blood relatively stably. From these results, it is expected that the dipeptides and tripeptides having the sequences Xaa-Pro and Xaa-Pro-Pro, respectively, exhibit high absorbability and digestion resistance in living organism. Thus it is assumed that Gln-Pro, Met-Pro, and Ser-Pro-Pro of the present invention also have high absorbability and digestion resistance in living organism.

**Claims**

1. A peptide having Pro at a carboxyl terminal, selected from the group consisting of Gln-Pro, Met-Pro, and Ser-Pro-Pro, or a salt thereof, for use in medicine.

**2.** The peptide, or salt thereof, of claim 1 for use as an angiotensin converting enzyme inhibitor.

**3.** The peptide, or salt thereof, of claim 1 for use in a functional food having hypotensive effect.

**4.** Use of a peptide having Pro at a carboxyl terminal, selected from the group consisting of Gln-Pro, Met-Pro, and Ser-Pro-Pro, or a salt thereof, in the manufacture of a medicament or a functional food for reducing blood pressure.

**5.** The peptide, or salt thereof, of claims 1-3, wherein the peptide is Gln-Pro.

**6.** The peptide, or salt thereof, of claims 1-3, wherein the peptide is Met-Pro.

**7.** The peptide, or salt thereof, of claims 1-3, wherein the peptide is Ser-Pro-Pro.

**8.** The use of claim 4, wherein the peptide is Gln-Pro.

**9.** The use of claim 4, wherein the peptide is Met-Pro.

**10.** The use of claim 4, wherein the peptide is Ser-Pro-Pro.

**Patentansprüche**

**1.** Peptid, welches Pro am Carboxylende besitzt, ausgewählt aus der Gruppe bestehend aus Gln-Pro, Met-Pro und Ser-Pro-Pro, oder ein Salz davon zur Verwendung in der Medizin.

**2.** Peptid oder Salz davon nach Anspruch 1 zur Verwendung als Inhibitor des Angiotensin-konvertierenden Enzyms.

**3.** Peptid oder Salz davon nach Anspruch 1 zur Verwendung in einem funktionellen Lebensmittel, welches eine hypotonische Wirkung besitzt.

**4.** Verwendung eines Peptids, welches Pro am Carboxylende besitzt, ausgewählt aus der Gruppe bestehend aus Gln-Pro, Met-Pro und Ser-Pro-Pro, oder eines Salzes davon für die Herstellung eines Medikaments oder eines funktionellen Lebensmittels, um Blutdruck zu senken.

**5.** Peptid oder Salz davon nach einem der Ansprüche 1 bis 3, wobei das Peptid Gln-Pro ist.

**6.** Peptid oder Salz davon nach einem der Ansprüche 1 bis 3, wobei das Peptid Met-Pro ist.

**7.** Peptid oder Salz davon nach einem der Ansprüche 1 bis 3, wobei das Peptid Ser-Pro-Pro ist.

**8.** Verwendung nach Anspruch 4, wobei das Peptid Gln-Pro ist.

**9.** Verwendung nach Anspruch 4, wobei das Peptid Met-Pro ist.

**10.** Verwendung nach Anspruch 4, wobei das Peptid Ser-Pro-Pro ist.

**Revendications**

**1.** Peptide ayant Pro à une extrémité terminale carboxyle, sélectionné dans le groupe constitué de Gln-Pro, Met-Pro et Ser-Pro-Pro ou sel de ce dernier, pour utilisation en médecine.

**2.** Peptide ou sel de ce dernier selon la revendication 1 pour utilisation comme inhibiteur de l'enzyme de conversion de l'angiotensine.

**3.** Peptide ou sel de ce dernier selon la revendication 1 pour utilisation dans un aliment fonctionnel ayant un effet hypotenseur.

**4.** Utilisation d'un peptide ayant Pro à une extrémité terminale carboxyle, sélectionné dans le groupe constitué de Gln-Pro, Met-Pro et Ser-Pro-Pro, ou d'un sel de ce dernier, dans la préparation d'un médicament ou d'un aliment fonctionnel destiné à abaisser la tension artérielle.

**5.** Peptide ou sel de ce dernier selon les revendications 1 à 3, où le peptide est Gln-Pro.

**6.** Peptide ou sel de ce dernier selon les revendications 1 à 3, où le peptide est Met-Pro.

**7.** Peptide ou sel de ce dernier selon les revendications 1 à 3, où le peptide est Ser-Pro-Pro.

**8.** Utilisation selon la revendication 4, où le peptide est Gln-Pro.

**9.** Utilisation selon la revendication 4, où le peptide est Met-Pro.

**10.** Utilisation selon la revendication 4, où le peptide est Ser-Pro-Pro.

# Fig. 1

**Absorption of Peptides into Blood**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2062828 A
- JP 3120225 A
- JP 6040944 A
- JP 5252979 A

**Non-patent literature cited in the description**

- **Cheung et al.** Binding of peptide substrates and inhibitors of angiotensin-converting enzyme. Importance of the COOH-terminal dipeptide sequence. *J Biol Chem.,* 25 January 1980, vol. 255 (2), 401-7
- **Harris et al.** Dipeptide-hydroxamates are good inhibitors of the angiotensin I-converting enzyme. *Biochem Biophys Res Commun,* 31 October 1983, vol. 116 (2), 394-9